(19) Europäisches Patentamt
European Patent Office
Office européen des brevets

(11) **EP 4 501 902 A1**

(12) **EUROPEAN PATENT APPLICATION**
published in accordance with Art. 153(4) EPC

(43) Date of publication:
**05.02.2025 Bulletin 2025/06**

(21) Application number: 23774525.2

(22) Date of filing: **08.03.2023**

(51) International Patent Classification (IPC):
***C07C 67/54*** (2006.01)     ***C07C 69/54*** (2006.01)
***C07D 303/16*** (2006.01)

(52) Cooperative Patent Classification (CPC):
(C-Sets available)
**C07C 67/54; C07D 303/16**     (Cont.)

(86) International application number:
**PCT/JP2023/008707**

(87) International publication number:
**WO 2023/181926 (28.09.2023 Gazette 2023/39)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **25.03.2022 JP 2022050007**

(71) Applicant: **MITSUBISHI GAS CHEMICAL
COMPANY, INC.**
**Chiyoda-ku**
**Tokyo 100-8324 (JP)**

(72) Inventors:
• **YURI, Michihiro**
  **Niigata-shi, Niigata 950-3121 (JP)**
• **SUZUKI, Kouji**
  **Hiratsuka-shi, Kanagawa 254-0016 (JP)**
• **YAMAGUCHI, Yu**
  **Niigata-shi, Niigata 950-3121 (JP)**
• **SUGITA, Masaki**
  **Niigata-shi, Niigata 950-3112 (JP)**
• **SUGANO, Yuuichi**
  **Tokyo 100-8324 (JP)**

(74) Representative: **Vossius & Partner
Patentanwälte Rechtsanwälte mbB
Siebertstrasse 3
81675 München (DE)**

(54) **METHOD FOR PURIFYING (METH)ACRYLIC ACID MONOMER AND/OR (METH)ACRYLIC ACID ESTER MONOMER**

(57)     According to one embodiment, provided is a method for purifying a (meth)acrylic acid monomer and/or a (meth)acrylic acid ester monomer, comprising purifying a composition containing a (meth)acrylic acid monomer and/or a (meth)acrylic acid ester monomer by distillation, wherein from the beginning to the end of the distillation, oxygen is supplied, so that the molar ratio of oxygen to vapor derived from the (meth)acrylic acid monomer and/or the (meth)acrylic acid ester monomer in gas phase components in a distillation still is maintained at $1.50 \times 10^{-3}$ or more.

EP 4 501 902 A1

(52) Cooperative Patent Classification (CPC): (Cont.)

C-Sets
**C07C 67/54, C07C 69/54**

## Description

Technical Field

The present invention relates to a method for purifying a (meth)acrylic acid monomer and/or a (meth)acrylic acid ester monomer. In addition, the present invention also relates to a method for producing a purified (meth)acrylic acid monomer and/or a purified (meth)acrylic acid ester monomer, comprising the aforementioned purification method.

Background Art

A (meth)acrylic acid monomer and a (meth)acrylic acid ester monomer (hereinafter collectively referred to as a "(meth)acryl monomer" at times) are polymerizable compounds having a carbon-carbon double bond. The polymerization reaction thereof can be induced by utilizing light, heat, radicals, ionic substances, metal complex salts, and the like. Utilizing their property by which the polymerization reaction easily progresses, in particular, the (meth)acrylic acid monomer is used in the production of water-soluble polymers, the production of copolymers with other vinyl compounds, the production of ion-exchange resins, etc. On the other hand, since the polymerization reaction occurs only by leaving them for a long time, it is a problem that the polymerization reaction occurs even when polymerization is not desired, for example, when the (meth)acryl monomer itself is used as a product.

Upon the production of the (meth)acryl monomer, the purification process is generally carried out by distillation. In distillation, since a high-purity (meth)acryl monomer is handled under heated conditions, the polymerization reaction of the (meth)acryl monomer is likely to occur. In particular, such polymerization reactions are likely to occur inside a distillation column and a condenser, in which vapor tends to stay. When the device is blocked by the occurrence of the polymerization reaction, it is necessary to terminate the operation and remove the polymerized product. This requires extra labor, and as a result, it leads to a decrease in production efficiency.

In order to solve this problem, various efforts have been made so far. For example, the use of a polymerization inhibitor has been used. At present, the use of such a polymerization inhibitor is common in the distillation and purification of the (meth)acryl monomer. However, the use of a polymerization inhibitor has caused a new problem. In order to suppress polymerization reactions in the distillation column and the condenser, it is desired to use a polymerization inhibitor that vaporizes together with the (meth)acryl monomer. However, when such a polymerization inhibitor is used, the polymerization inhibitor would be mixed into the (meth)acryl monomer product obtained by purification. In other words, if a large amount of such a polymerization inhibitor is used to suppress the polymerization reaction, problems such as a reduction in the purity of the (meth)acryl monomer product and coloration caused by the polymerization inhibitor may newly arise.

In addition to the use of a polymerization inhibitor, there are other methods of suppressing polymerization reactions by supplying gases having polymerization inhibitory action, such as nitrogen oxides (e.g., nitric oxide, nitrogen dioxide, etc.) and oxygen, to the inside of the distillation column (Patent Literature 1). However, nitrogen oxide may alter the (meth)acryl monomer and the polymerization inhibitor in the system. In addition, nitric oxide is difficult to be handled because it easily reacts with oxygen in the air to form nitrogen dioxide. Oxygen also has polymerization inhibitory action, but its behavior is extremely complicated, and thus, the effective use of oxygen for suppression of the polymerization reaction has not yet been achieved under the current circumstances.

Moreover, a method of stabilizing the acryl monomer using a metal ion sequestering agent and suppressing the polymerization reaction has also been known (Patent Literature 2).

Citation List

Patent Literature

    Patent Literature 1: JP Patent Publication (Kohyo) No. 2003-532631 A
    Patent Literature 2: JP Patent Publication (Kohyo) No. 2004-513089 A

Summary of Invention

Technical Problem

It is an object of the present invention to provide a method for purifying a (meth)acrylic acid monomer and/or a (meth)acrylic acid ester monomer, while suppressing a polymerization reaction.

Solution to Problem

As mentioned above, in the purification of (meth)acryl monomers, the polymerization inhibitory action of oxygen has not been fully utilized under the current circumstances. The reason therefor is that oxygen in the ground state is a biradical, which directly reacts with monomers and also reacts with radicals to form peroxy radicals, thereby making it difficult to control the reaction. In addition, hydrogen is extracted by peroxy radicals, and since peroxides generated by the recombination of oxygen and radicals are decomposed to form radicals, they may act as polymerization initiators. Thus, while oxygen is difficult to be controlled, the oxygen is inexpensive and does not contaminate the product. Hence, the present inventors have thought that oxygen would be very useful in the purification of (meth)acryl monomers if its polymerization inhibitory action could be effectively utilized.

Accordingly, the present inventors have investigated the control of the ratio of the amount of oxygen to be supplied to the amount of a (meth)acryl monomer to be purified within a predetermined range in the distillation and purification of the (meth)acryl monomer. As a result, the present inventors have found that, according to the present method, the (meth)acryl monomer can be purified, while effectively suppressing the polymerization reaction.

Specifically, the present invention is, for example, as follows.

[1] A method for purifying a (meth)acrylic acid monomer and/or a (meth)acrylic acid ester monomer, comprising:

purifying a composition containing a (meth)acrylic acid monomer and/or a (meth)acrylic acid ester monomer by distillation, wherein

from the beginning to the end of the distillation, oxygen is supplied, so that the molar ratio of oxygen to vapor derived from the (meth)acrylic acid monomer and/or the (meth)acrylic acid ester monomer in gas phase components in a distillation still is maintained at $1.50 \times 10^{-3}$ or more, and

the molar ratio of the oxygen to the vapor derived from the (meth)acrylic acid monomer and/or the (meth)acrylic acid ester monomer is calculated according to the following Formula (1):

[Expression 1]

$$\frac{\text{Amount of air supplied per unit time (L/min)} \times \dfrac{\text{Oxygen concentration in air (\%)}}{100} \,/\, \text{Molar volume of air (L/mol)}}{\text{Amount of (meth)acryl monomer distilled per unit time (g/min)} \,/\, \text{Molar weight of (meth)acryl monomer}} \quad \text{... Formula (1)}$$

wherein the term "(meth)acryl monomer" means a (meth)acrylic acid monomer and/or a (meth)acrylic acid ester monomer to be purified.

[2] The method according to the above [1], wherein the molar ratio of the oxygen to the vapor derived from the (meth)acrylic acid monomer and/or the (meth)acrylic acid ester monomer is maintained at $1.67 \times 10^{-3}$ or more.

[3] The method according to the above [1] or [2], wherein the molar ratio of the oxygen to the vapor derived from the (meth)acrylic acid monomer and/or the (meth)acrylic acid ester monomer is maintained at $1.67 \times 10^{-3}$ to $8.00 \times 10^{-2}$.

[4] The method according to any one of the above [1] to [3], wherein the distillation is carried out in the absence of NO, $NO_2$ and a metal ion sequestering agent.

[5] The method according to any one of the above [1] to [4], wherein the distillation is carried out in the presence of one or more polymerization inhibitors selected from the group consisting of phenolic, amine-based, quinone-based, N-oxyradical-based, and nitroso-based polymerization inhibitors.

[6] The method according to the above [5], wherein the polymerization inhibitor comprises p-methoxyphenol.

[7] The method according to the above [5] or [6], wherein the polymerization inhibitor is comprised in a concentration of 10 to 50,000 ppm in a distilled mother liquor comprising the composition containing the (meth)acrylic acid monomer and/or the (meth)acrylic acid ester monomer.

[8] The method according to any one of the above [5] to [7], wherein the polymerization inhibitor is comprised in a concentration of 0 to 1,000 ppm in a distillate distilled from the distillation still.

[9] The method according to any one of the above [1] to [8], wherein the polymers of the (meth)acrylic acid and/or the (meth)acrylic acid ester are not substantially generated.

[9-1] The method according to any one of the above [1] to [9], wherein, in the method, the weight ratio (polymer/monomer) of the amount of the (meth)acryl polymer generated (kg) to the amount of the (meth)acryl monomer distilled (kg) during the distillation is $3.0 \times 10^{-4}$ or less.

[9-2] The method according to any one of the above [1] to [8], wherein, in the method, the polymers of the (meth)acrylic acid and/or the (meth)acrylic acid ester are not generated.

[10] The method according to any one of the above [1] to [9-2], wherein the (meth)acrylic acid and the (meth)acrylic acid ester are selected from the group consisting of methacrylic acid, methyl methacrylate, n-butyl methacrylate, isobutyl methacrylate, dimethylaminoethyl methacrylate, glycidyl methacrylate, 2-hydroxyethyl methacrylate, lauryl methacrylate, allyl methacrylate, 2-ethylhexyl methacrylate, cyclohexyl methacrylate, stearyl methacrylate, benzyl methacrylate, and diethylaminoethyl methacrylate.

[11] The method according to any one of the above [1] to [10], wherein the (meth)acrylic acid ester is glycidyl methacrylate.

[12] A method for producing a purified (meth)acrylic acid monomer and/or a purified (meth)acrylic acid ester monomer, comprising:

purifying a composition containing a (meth)acrylic acid monomer and/or a (meth)acrylic acid ester monomer by the method according to any one of the above [1] to [11], and

recovering the purified (meth)acrylic acid monomer and/or the purified (meth)acrylic acid ester monomer.

Advantageous Effects of Invention

According to the present invention, a method for purifying a (meth)acrylic acid monomer and/or a (meth)acrylic acid ester monomer, while suppressing a polymerization reaction, can be provided.

Description of Embodiments

Hereafter, the embodiments of the present invention will be described.

The method for purifying a (meth)acrylic acid monomer and/or a (meth)acrylic acid ester monomer according to one embodiment of the present invention comprises purifying a composition containing a (meth)acrylic acid monomer and/or a (meth)acrylic acid ester monomer by distillation. From the beginning to the end of the distillation, oxygen is supplied, so that the molar ratio of oxygen to vapor derived from the (meth)acrylic acid monomer and/or the (meth)acrylic acid ester monomer in gas phase components in a distillation still is maintained at $1.50 \times 10^{-3}$ or more. Herein, the above-described molar ratio of the oxygen to the vapor derived from the (meth)acrylic acid monomer and/or the (meth)acrylic acid ester monomer is calculated according to the following Formula (1):

[Expression 2]

$$\frac{\text{Amount of air supplied per unit time (L/min)} \times \dfrac{\text{Oxygen concentration in air (\%)}}{100} \Big/ \text{Molar volume of air (L/mol)}}{\text{Amount of (meth)acryl monomer distilled per unit time (g/min)} \Big/ \text{Molar weight of (meth)acryl monomer}} \quad \text{... Formula (1)}.$$

As mentioned above, purification of a (meth)acryl monomer by distillation has been problematic in that the polymerization reaction of the (meth)acryl monomer easily occurs, and in that the operation of removing a polymerized product is required, etc. In response to this problem, according to the embodiment of the present invention, by controlling the amount of oxygen supplied within a predetermined range, the (meth)acryl monomer can be purified, while the polymerization reaction is suppressed mainly in the gas phase portion of a distillation column. Accordingly, it is possible to avoid stopping the operation of a distillation device and removing the polymerized product during the process. In addition, according to the embodiment of the present invention, the polymerization reaction can be suppressed without using a large amount of polymerization inhibitor, since the polymerization reaction inhibitory action of oxygen can be effectively utilized. Therefore, problems such as coloration and a reduction in the purity of the product after purification due to the polymerization inhibitor are unlikely to occur. Thus, the method according to the embodiment of the present invention is a simple and efficient purification method, and it is also an economically advantageous method because it uses inexpensive oxygen. As mentioned above, conventionally, there has been a problem of difficulty in controlling the reaction when oxygen is used, but the method according to the embodiment of the present invention hardly causes such a problem regarding the control of the reaction. This is considered to be because of the fact that the amount of a peroxide generated is small, and that the 10-hour half-life temperature of dialkyl peroxide or hydroperoxide, which is considered to be generated in the distillation and purification process using oxygen, is 100°C or higher, etc.

Furthermore, according to the embodiment of the present invention, although a third component having polymerization reaction inhibitory action (e.g., nitrogen oxide such as NO or $NO_2$, a metal ion sequestering agent, etc.) other than oxygen and the polymerization inhibitor (hereinafter also referred to as a "polymerization reaction inhibitory component") is not used, sufficient polymerization reaction inhibitory effects can be obtained. Therefore, if the method according to the embodiment is carried out without using these components, the method can be carried out more easily because it is no longer necessary to pay attention to the handling of these components. In addition, since impurities caused by the third component are not mixed into a product obtained after purification, the purity of the product is not reduced. Furthermore, the cost of the third component can also be reduced.

Hereafter, individual steps and components applied in the method of the present invention will be described, successively.

The method according to the present embodiment can be used for purification of any substance, as long as the substance is generally classified into (meth)acrylic acid and (meth)acrylic acid ester. Thus, the (meth)acrylic acid and the (meth)acrylic acid ester to be purified are not particularly limited, but are preferably selected from the group consisting of methacrylic acid, methyl methacrylate, n-butyl methacrylate, isobutyl methacrylate, dimethylaminoethyl methacrylate, glycidyl methacrylate, 2-hydroxyethyl methacrylate, lauryl methacrylate, allyl methacrylate, 2-ethylhexyl methacrylate, cyclohexyl methacrylate, stearyl methacrylate, benzyl methacrylate, and diethylaminoethyl methacrylate. The (meth) acrylic acid and the (meth)acrylic acid ester are more preferably selected from the group consisting of methacrylic acid, methyl methacrylate, n-butyl methacrylate, isobutyl methacrylate, dimethylaminoethyl methacrylate, glycidyl methacrylate and 2-hydroxyethyl methacrylate. The (meth)acrylic acid ester is particularly preferably glycidyl methacrylate (hereinafter, the glycidyl methacrylate is also referred to as GMA). The composition to be purified may comprise one or two or more (meth)acrylic acid monomers and/or (meth)acrylic acid ester monomers. It is to be noted that the term "(meth)acrylic acid" means methacrylic acid and/or acrylic acid, and the term "(meth)acrylic acid ester" means methacrylic acid ester and/or acrylic acid ester. Components other than the (meth)acryl monomer comprised in the composition to be purified may include, but are not limited to, excess raw materials and reaction by-products.

Purification is carried out by distillation. Distillation can be carried out based on common methods used in the present field, and the apparatus used is also an apparatus that is commonly used in the present field. The method according to the embodiment of the present invention is not restricted by the distillation method and the distillation apparatus. For example, all of batch distillation, continuous distillation, single distillation, and rectification can be used, and also, the method according to the embodiment of the present invention is not restricted in any way by the structure of the distillation equipment (the location of air blowing, a heating method, the structure of a vapor line, the position of a condenser, etc.). The distillation method and the distillation device (equipment structure) can be selected, as appropriate, by those skilled in the art according to the purpose.

For example, distillation can be carried out under normal pressure or reduced pressure and by heating to 40°C to 150°C.

From the beginning to the end of the distillation, the amount of oxygen supplied is controlled within a predetermined range. Specifically, from the beginning to the end of the distillation, oxygen is supplied, so that the molar ratio of oxygen to vapor derived from the (meth)acrylic acid monomer and/or the (meth)acrylic acid ester monomer in gas phase components in a distillation still is maintained at $1.50 \times 10^{-3}$ or more. Herein, the beginning of the distillation means the time point at which the supply of oxygen is started, after the system has been heated and evaporation of the (meth)acryl monomer has been started. The end of the distillation may be any time point, but it means the time point at which the oxygen supply is terminated.

Herein, the molar ratio of the oxygen to the vapor derived from the (meth)acryl monomer in the gas phase components is calculated according to the following Formula (1):

[Expression 3]

$$\frac{\text{Amount of air supplied per unit time (L/min)} \times \dfrac{\text{Oxygen concentration in air (\%)}}{100} / \text{Molar volume of air (L/mol)}}{\text{Amount of (meth)acryl monomer distilled per unit time (g/min)} / \text{Molar weight of (meth)acryl monomer}} \quad \text{... Formula (1)}.$$

The "molar ratio of oxygen to vapor derived from (meth)acrylic acid and/or (meth)acrylic acid ester in gas phase components in a distillation still" is, in other words, the molar ratio between the oxygen supplied per unit time and the (meth) acryl monomer distilled per unit time. The amount of air supplied per unit time and the amount of monomer distilled per unit time can be regulated, as appropriate, by changing the settings of the equipment, etc. The oxygen concentration in the air and the molar volume of the air are different depending on the air used. In the Examples described later, the air used was an ideal gas (molar volume: 24.04 L/mol) at 20°C having an oxygen concentration of 21 (v/v)%. However, the air used is not limited thereto, and the molar ratio can be calculated by introducing the values of an oxygen concentration (% by volume) and the molar volume of air into Formula (1) according to the air used. The amount of the monomer distilled (g/min) per unit time is also referred to as a "monomer distillation rate," which means the weight (g) of the monomer distilled per unit time (min). The monomer distillation rate can be calculated by measuring the volume (mL) of a (meth)acryl monomer distilled per unit time, and then considering the specific gravity (g/mL) of the (meth)acryl monomer.

The molar ratio calculated by the above Formula (1) is preferably $1.50 \times 10^{-3}$ or more, more preferably $1.67 \times$

$10^{-3}$ or more, further preferably $1.50 \times 10^{-3}$ to $8.00 \times 10^{-2}$, and particularly preferably $1.67 \times 10^{-3}$ to $8.00 \times 10^{-2}$. Otherwise, the above-described molar ratio may be in the range of $1.94 \times 10^{-3}$ to $8.00 \times 10^{-2}$, $2.29 \times 10^{-3}$ to $8.00 \times 10^{-2}$, or $2.78 \times 10^{-3}$ to $8.00 \times 10^{-2}$. Otherwise, the above-described molar ratio may also be in the range of $1.50 \times 10^{-3}$ to $1.00 \times 10^{-2}$, $1.50 \times 10^{-3}$ to $5.00 \times 10^{-3}$, or $1.50 \times 10^{-3}$ to $3.00 \times 10^{-3}$. By controlling the amount of oxygen supplied within such a range, the (meth)acryl monomer can be purified, while suppressing the polymerization reaction mainly in the gas phase portion of a distillation column. Moreover, by setting the amount of oxygen supplied within the above-described range (below the upper limit value), the amount of gas to be treated does not become excessive, which is a further advantage also from the viewpoints of safety and economy. If the amount of oxygen supplied is set within such a range, the distillation column does not need to be excessively enlarged, either. The amount of oxygen supplied can be easily controlled by changing the settings of the equipment while checking the amount of (meth)acryl monomer distilled.

When a (meth)acryl monomer is purified by the method according to the embodiment of the present invention, the amount of a (meth)acryl polymer generated becomes small because the polymerization reaction is suppressed. Herein, the term "(meth)acryl polymer" means a polymer generated by polymerization of a (meth)acrylic acid monomer and/or a (meth)acrylic acid ester monomer. In a preferred embodiment, the (meth)acryl polymer is not substantially generated in the distillation process. The phrase "be not substantially generated" includes not only a case where no (meth)acryl polymer is generated, but also a case where a (meth)acryl polymer is generated in an amount that does not affect the distillation process. The phrase "in an amount that does not affect the distillation process" means, for example, the amount in which it is not necessary to stop the operation during the distillation process and to remove the generated polymer (i.e., the amount that allows continuous operation until the end of distillation). For example, the ratio between the amount of (meth)acryl polymer generated (kg) and the amount of (meth)acryl monomer distilled (kg) during distillation (i.e., weight ratio: polymer/monomer) may be $3.0 \times 10^{-4}$ or less (0 to $3.0 \times 10^{-4}$), or $1.0 \times 10^{-4}$ or less (0 to $1.0 \times 10^{-4}$).

Distillation may be carried out in the presence of a polymerization inhibitor. The polymerization inhibitor is not limited, and can be selected, as appropriate, from among those used in the present field. Examples of such a polymerization inhibitor may include phenolic, amine-based, quinone-based, N-oxyradical-based, and nitroso-based polymerization inhibitors. Examples of the phenolic polymerization inhibitor may include p-methoxyphenol, hydroquinone, cresol, tert-butylhydroquinone, 2,5-di-tert-butylhydroquinone, 2,4-dimethyl-6-tert-butylphenol, 2,6-di-tert-butylphenol, 2,6-di-tert-butyl-p-cresol, 4-tert-butylpyrocatechol, 2,2'-methylenebis(4-methyl-6-tert-butylphenol), 2,2'-methylene-bis(4-ethyl-6-tert-butylphenol), and 4,4'-thiobis(3-methyl-6-tert-butylphenol). Examples of the amine-based polymerization inhibitor may include phenothiazine, p-phenylenediamine, 4-aminodiphenylamine, N,N'-diphenyl-p-phenylenediamine, N-i-propyl-N'-phenyl-p-phenylenediamine, N-(1,3-dimethylbutyl)-N'-phenyl-p-phenylenediamine, N,N'-di-2-naphthyl-p-phenylenediamine, diphenylamine, N-phenyl-β-naphthylamine, 4,4'-dicumyl-diphenylamine, and 4,4'-dioctyl diphenylamine. Examples of the quinone-based polymerization inhibitor may include 1,4-benzoquinone, and 2-tert-butyl-1,4-benzoquinone. Examples of the N-oxyradical-based polymerization inhibitor may include 2,2,6,6-tetramethylpiperidine 1-oxyl free radical, 4-oxo-2,2,6,6-tetramethylpiperidine 1-oxyl free radical, 4-amino-2,2,6,6-tetramethylpiperidine 1-oxyl free radical, 4-hydroxy-2,2,6,6-tetramethylpiperidine 1-oxyl free radical, 4-methoxy-2,2,6,6-tetramethylpiperidine 1-oxyl free radical, and 4-acetamido-2,2,6,6-tetramethylpiperidine 1-oxyl free radical. Examples of the nitroso-based polymerization inhibitor may include N-nitrosodiphenylamine, N-nitrosophenylnaphthylamine, N-nitrosodinaphthylamine, p-nitrosophenol, nitrosobenzene, p-nitrosodiphenylamine, and α-nitroso-β-naphthol. The used polymerization inhibitor may be one type, or may also be two or more types.

Among these, p-methoxyphenol, hydroquinone, cresol, tert-butylhydroquinone, 2,5-di-tert-butylhydroquinone, 2,4-dimethyl-6-tert-butylphenol, 2,6-di-tert-butylphenol, 2,6-di-tert-butyl-p-cresol, and 4-tert-butylpyrocatechol are preferable. From the viewpoint of economy, p-methoxyphenol is particularly preferable. In addition, when p-methoxyphenol is used in the method according to the embodiment of the present invention, desired effects are obtained with a particularly small amount, and the amount of p-methoxyphenol contained as an impurity in the distillate after distillation becomes small. Therefore, problems such as coloration and a reduction in the purity of the product after purification due to the polymerization inhibitor are less likely to occur, and also, it is advantageous in that there is no need for performing a further purification process, etc.

The polymerization inhibitor may be contained in a distilled mother liquor (a mixed solution before heating) comprising a composition containing a (meth)acryl monomer in a concentration of, for example, 10 to 50,000 ppm, 10 to 30,000 ppm, 50 to 30,000 ppm, or 50 to 10,000 ppm. The amount of the polymerization inhibitor is not particularly limited, but considering the aforementioned various problems caused by the polymerization inhibitor, the amount of the polymerization inhibitor is preferably small. Since the polymerization reaction inhibitory action of oxygen can be effectively utilized according to the method of the embodiment of the present invention, the polymerization reaction can be suppressed, even if the polymerization inhibitor is not used in a large amount. When the method according to the embodiment of the present invention is carried out, the amount of a polymerization inhibitor as an impurity in the distillate after distillation can be suppressed in conjunction with the small amount of the polymerization inhibitor used. For example, the distillate distilled from a distillation still contains a polymerization inhibitor in a concentration of 0 to 1,000 ppm, 0 to 100 ppm, or 0 to 10 ppm. In a preferred embodiment, the polymerization inhibitor is not contained in the distillate distilled from the distillation still.

The method according to the embodiment of the present invention can be carried out in the absence of nitrogen oxides (NO, $NO_2$, etc.) and a metal ion sequestering agent, which have polymerization reaction inhibitory action. As mentioned above, since the disadvantages of using, in particular, NO, $NO_2$ or a metal ion sequestering agent are large, the advantage of obtaining sufficient polymerization reaction inhibitory effects even if distillation is carried out in the absence of these substances is extremely significant.

Furthermore, the method according to the embodiment of the present invention can be carried out using only oxygen and the aforementioned polymerization inhibitor as components having polymerization reaction inhibitory action. In other words, the method according to the embodiment of the present invention can be carried out in the absence of polymerization reaction inhibitory components except for oxygen and the polymerization inhibitor. Examples of such polymerization reaction inhibitory components may include nitrogen oxides such as NO and $NO_2$, and metal ion sequestering agents. According to the method of the embodiment of the present invention, sufficient polymerization reaction inhibitory effects can be obtained, although these polymerization reaction inhibitory components are not used.

According to another embodiment of the present invention, a method for producing a (meth)acryl monomer, comprising the above-described purification method, is provided. The present production method comprises purifying a composition containing a (meth)acryl monomer by the aforementioned method, and recovering the purified (meth)acryl monomer. The method of recovering the purified (meth)acryl monomer is not particularly limited, and a method used in the present technical field can be used.

Examples

Hereinafter, the present invention will be described in more detail in the following examples. However, the content of the present invention is not limited by these examples.

< Operation Method >

To a 500-mL flask equipped with a distillation column (size: 30 mmΦ × 200 mm; filler: DIXON Packing 6 mm, SUS316 manufactured by TO-TOKU ENGINEERING CO., LTD.), 300 g of glycidyl methacrylate (GMA) and 0.3 g of 2,2'-methylene-bis(4-methyl-6-tert-butylphenol) were added, so as to prepare a distilled mother liquor. To this distilled mother liquor, a predetermined amount of p-methoxyphenol was added. Thereafter, while the pressure in the distillation column was reduced to 1.6 kPaA, the oil bath was heated, and evaporation of glycidyl methacrylate was started. While continuing the heating of the oil bath, a predetermined amount of air was supplied. A total reflux operation was carried out for 6 hours, while controlling so that the glycidyl methacrylate was distilled at a constant rate.

After completion of the operation, a glycidyl methacrylate-containing solution that was distilled into a distillate pool was collected, and the concentration of p-methoxyphenol in the solution was then analyzed. Thereafter, the weight of the glycidyl methacrylate polymer generated in the distillation column was measured. Specifically, the distillation column was washed with methanol and was then dried, and the weights of the distillation columns before and after distillation were compared with each other, and the difference was determined to be the weight of the generated polymer.

It is to be noted that, in each example, the molar ratio of the oxygen to the vapor derived from glycidyl methacrylate in the gas phase components in the distillation column was controlled to be a predetermined value by regulating the air supply rate (the amount of air supplied per unit time) and the distillation rate of glycidyl methacrylate. The distillation rate of glycidyl methacrylate can be controlled by regulating the heating temperature of the oil bath. Herein, the distillation rate of glycidyl methacrylate (g/min) (i.e., the amount of GMA distilled per unit time (g/min) is the weight (g) of glycidyl methacrylate distilled per unit time) was calculated by measuring the volume (mL) of glycidyl methacrylate distilled per unit time and considering the specific gravity of glycidyl methacrylate (1.074 (g/mL).

The molar ratio of the oxygen to the vapor derived from glycidyl methacrylate in the gas phase components in the distillation column was calculated according to the following Formula (1) (hereinafter also referred to as a "molar ratio $[O_2/GMA]$"):

[Expression 4]

$$\frac{\text{Amount of air supplied per unit time (L/min)} \times \frac{\text{Oxygen concentration in air (\%)}}{100} / \text{Molar volume of air (L/mol)}}{\text{Amount of (meth)acryl monomer distilled per unit time (g/min)} / \text{Molar weight of (meth)acryl monomer}} \quad ...\text{Formula (1)}.$$

Herein, the oxygen concentration in the supplied air was 21 (v/v)%, and it was an ideal gas (24.04 L/mol) at 20°C. Accordingly, "the amount of air supplied per unit time (L/min) × 0.21" is defined to be the amount of oxygen supplied per unit time (L/min).

(Example 1)

Distillation was carried out according to the aforementioned operation method.

In Example 1, the concentration of p-methoxyphenol in the distilled mother liquor was set to be 66 ppm, the amount of air supplied per unit time was set to be 10 mL/min (the amount of oxygen supplied per unit time was 10 mL/min $\times$ 0.21 = 2.1 mL/min), and the distillation rate of glycidyl methacrylate was set to be 4.5 g/min. The molar ratio [$O_2$/GMA] calculated using the above Formula (1) was $2.78 \times 10^{-3}$. Herein, the value of the molecular weight of glycidyl methacrylate was set to be 142.15.

(Examples 2 to 5 and Comparative Example 1)

In Examples 2 to 5 and Comparative Example 1, distillation was carried out in the same manner as that of Example 1 under the conditions shown in the following Table 1.

[Table 1]

| | Ex. 1 | Ex. 2 | Ex. 3 | Ex. 4 | Ex. 5 | Comp. Ex. 1 |
|---|---|---|---|---|---|---|
| Amount of air supplied (mL/min) | 10 | 10 | 10 | 10 | 8 | 8 |
| GMA distillation rate (g/min) | 4.5 | 5.4 | 6.4 | 7.4 | 5.9 | 7.4 |
| p-Methoxyphenol (ppm) | 66 | 9741 | 9741 | 9741 | 9741 | 9741 |
| Molar ratio [$O_2$/GMA] | $2.78 \times 10^{-3}$ | $2.29 \times 10^{-3}$ | $1.94 \times 10^{-3}$ | $1.67 \times 10^{-3}$ | $1.67 \times 10^{-3}$ | $1.34 \times 10^{-3}$ |

After completion of the operation, according to the aforementioned methods, the concentration of p-methoxyphenol contained in the distillate and the weight of a glycidyl methacrylate polymer generated in the distillation process were measured. In addition, the ratio (weight ratio: polymer/monomer) between the amount of the glycidyl methacrylate polymer generated (kg) and the amount of the glycidyl methacrylate monomer distilled during the distillation (kg) was calculated. The results are shown in the following Table 2.

[Table 2]

| | Ex. 1 | Ex. 2 | Ex. 3 | Ex. 4 | Ex. 5 | Comp. Ex. 1 |
|---|---|---|---|---|---|---|
| Amount of polymer generated (g) | 0 | 0 | 0 | 0 | 0 | 0.75 |
| p-Methoxyphenol concentration in distillate (ppm) | 0 | 4.6 | 7.2 | 7.4 | 3.6 | 7.2 |
| Weight ratio (polymer/monomer) | 0 | 0 | 0 | 0 | 0 | $3.4 \times 10^{-4}$ |

Thus, according to the method of the Examples, the amount of the glycidyl methacrylate polymer generated is small. That is to say, it can be said that the glycidyl methacrylate monomer could be purified, while suppressing the polymerization reaction. Moreover, according to the above-described Examples, the above-described effects could be achieved without using components having polymerization reaction inhibitory effects except for oxygen and the polymerization inhibitor. Furthermore, since the amount of the polymerization inhibitor used (in the Examples, p-methoxyphenol) is small, the amount of the polymerization inhibitor contained in the distillate is also small. Therefore, it can be said that it is unlikely to cause problems such as coloration and a reduction in the purity of the product after purification due to polymerization inhibitors.

Several embodiments of the present invention have been described. These embodiments are presented as examples and are not intended to limit the scope of the invention. These novel embodiments can be carried out in various other forms, and various omissions, replacements, and modifications can be made in a range that does not depart from the gist of the invention. These embodiments and variations thereof are included in the scope and gist of the invention, as well as in the scope of the invention and its equivalents recited in the claims.

**Claims**

1. A method for purifying a (meth)acrylic acid monomer and/or a (meth)acrylic acid ester monomer, comprising:

   purifying a composition containing a (meth)acrylic acid monomer and/or a (meth)acrylic acid ester monomer by distillation, wherein
   from the beginning to the end of the distillation, oxygen is supplied, so that the molar ratio of oxygen to vapor derived from the (meth)acrylic acid monomer and/or the (meth)acrylic acid ester monomer in gas phase components in a distillation still is maintained at $1.50 \times 10^{-3}$ or more, and
   the molar ratio of the oxygen to the vapor derived from the (meth)acrylic acid monomer and/or the (meth)acrylic acid ester monomer is calculated according to the following Formula (1):

   [Expression 1]

   $$\frac{\text{Amount of air supplied per unit time (L/min)} \times \frac{\text{Oxygen concentration in air (\%)}}{100} / \text{Molar volume of air (L/mol)}}{\text{Amount of (meth)acryl monomer distilled per unit time (g/min)} / \text{Molar weight of (meth)acryl monomer}} \cdots \text{Formula (1).}$$

2. The method according to claim 1, wherein the molar ratio of the oxygen to the vapor derived from the (meth)acrylic acid monomer and/or the (meth)acrylic acid ester monomer is maintained at $1.67 \times 10^{-3}$ or more.

3. The method according to claim 1 or 2, wherein the molar ratio of the oxygen to the vapor derived from the (meth)acrylic acid monomer and/or the (meth)acrylic acid ester monomer is maintained at $1.67 \times 10^{-3}$ to $8.00 \times 10^{-2}$.

4. The method according to any one of claims 1 to 3, wherein the distillation is carried out in the absence of NO, $NO_2$ and a metal ion sequestering agent.

5. The method according to any one of claims 1 to 4, wherein the distillation is carried out in the presence of one or more polymerization inhibitors selected from the group consisting of phenolic, amine-based, quinone-based, N-oxyradical-based, and nitroso-based polymerization inhibitors.

6. The method according to claim 5, wherein the polymerization inhibitor comprises p-methoxyphenol.

7. The method according to claim 5 or 6, wherein the polymerization inhibitor is comprised in a concentration of 10 to 50,000 ppm in a distilled mother liquor comprising the composition containing the (meth)acrylic acid monomer and/or the (meth)acrylic acid ester monomer.

8. The method according to any one of claims 5 to 7, wherein the polymerization inhibitor is comprised in a concentration of 0 to 1,000 ppm in a distillate distilled from the distillation still.

9. The method according to any one of claims 1 to 8, wherein the polymers of the (meth)acrylic acid and/or the (meth)acrylic acid ester are not substantially generated.

10. The method according to any one of claims 1 to 9, wherein the (meth)acrylic acid and the (meth)acrylic acid ester are selected from the group consisting of methacrylic acid, methyl methacrylate, n-butyl methacrylate, isobutyl methacrylate, dimethylaminoethyl methacrylate, glycidyl methacrylate, 2-hydroxyethyl methacrylate, lauryl methacrylate, allyl methacrylate, 2-ethylhexyl methacrylate, cyclohexyl methacrylate, stearyl methacrylate, benzyl methacrylate, and diethylaminoethyl methacrylate.

11. The method according to any one of claims 1 to 10, wherein the (meth)acrylic acid ester is glycidyl methacrylate.

12. A method for producing a purified (meth)acrylic acid monomer and/or a purified (meth)acrylic acid ester monomer, comprising:

    purifying a composition containing a (meth)acrylic acid monomer and/or a (meth)acrylic acid ester monomer by the method according to any one of claims 1 to 11, and
    recovering the purified (meth)acrylic acid monomer and/or the purified (meth)acrylic acid ester monomer.

## INTERNATIONAL SEARCH REPORT

| International application No. |
|---|
| **PCT/JP2023/008707** |

### A. CLASSIFICATION OF SUBJECT MATTER

*C07C 67/54*(2006.01)i; *C07C 69/54*(2006.01)i; *C07D 303/16*(2006.01)i
FI: C07C67/54; C07C69/54 Z; C07D303/16

According to International Patent Classification (IPC) or to both national classification and IPC

### B. FIELDS SEARCHED

Minimum documentation searched (classification system followed by classification symbols)

C07C67/00; C07C69/00; C07D303/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X | JP 2005-232008 A (MITSUBISHI CHEMICAL CORP.) 02 September 2005 (2005-09-02) claim 1, paragraph [0050] | 1-12 |
| X | JP 2005-179352 A (MITSUBISHI CHEMICAL CORP.) 07 July 2005 (2005-07-07) claims 1-17 | 1-12 |
| X | JP 2004-513089 A (ATOFINA) 30 April 2004 (2004-04-30) claims 1-10 | 1-12 |
| X | WO 2005/102984 A1 (DAIKIN INDUSTRIES, LTD.) 03 November 2005 (2005-11-03) claims 1-8 | 1-12 |
| X | JP 2000-300901 A (NIPPON SHOKUBAI CO., LTD.) 31 October 2000 (2000-10-31) claims 1-3, examples 1, 2 | 1-12 |
| X | JP 2001-114705 A (NIPPON SHOKUBAI CO., LTD.) 24 April 2001 (2001-04-24) example 3 | 1-12 |

☐ Further documents are listed in the continuation of Box C.       ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **22 May 2023** | **30 May 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2023/008707**

| Patent document cited in search report | | | Publication date (day/month/year) | Patent family member(s) | | | Publication date (day/month/year) |
|---|---|---|---|---|---|---|---|
| JP | 2005-232008 | A | 02 September 2005 | US claims 1-5 | 2004/0222077 | A1 | |
| | | | | WO | 2003/018162 | A1 | |
| | | | | CN | 1550249 | A | |
| JP | 2005-179352 | A | 07 July 2005 | US schemes 1-17 | 2006/0205979 | A1 | |
| | | | | WO | 2005/051883 | A1 | |
| | | | | EP | 1688407 | A1 | |
| | | | | CN | 1697818 | A | |
| JP | 2004-513089 | A | 30 April 2004 | US claims 1-10 | 2006/0205979 | A1 | |
| | | | | WO | 2005/051883 | A1 | |
| | | | | EP | 1688407 | A1 | |
| | | | | CN | 1697818 | A | |
| WO | 2005/102984 | A1 | 03 November 2005 | US claims 1-8 | 2007/0232766 | A1 | |
| | | | | WO | 2005/102984 | A1 | |
| | | | | EP | 1757578 | A1 | |
| | | | | CN | 1946669 | A | |
| JP | 2000-300901 | A | 31 October 2000 | US claims 1-3, examples 1, 2 | 6627047 | B1 | |
| | | | | EP | 1046416 | A2 | |
| | | | | CN | 1273136 | A | |
| JP | 2001-114705 | A | 24 April 2001 | US example 3 | 6454541 | B1 | |
| | | | | EP | 1092874 | A2 | |
| | | | | CN | 1292471 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003532631 A
- JP 2004513089 A